Europäisches Patentamt

European Patent Office

Office européen des brevets

(19)

(11) Publication number: **0 183 441**

**A1**

(12) # EUROPEAN PATENT APPLICATION

(21) Application number: **85308287.3**

(22) Date of filing: **14.11.85**

(51) Int. Cl.⁴: **A 61 L 2/06**
**//A61L15/03, A61K9/70**

(30) Priority: **15.11.84 GB 8428899**

(43) Date of publication of application:
**04.06.86 Bulletin 86/23**

(84) Designated Contracting States:
**AT BE CH DE FR GB IT LI LU NL SE**

(71) Applicant: **SCHERING CHEMICALS LIMITED**
**The Brow Burgess Hill**
**West Sussex RH15 9NE(GB)**

(72) Inventor: **Shaw, Robert**
**50 Duke Street**
**Formby Merseyside(GB)**

(74) Representative: **Waldman, Ralph David**
**Industrial Property Department FBC Limited Chesterford**
**Park Research Station**
**Saffron Walden, Essex CB10 1XL(GB)**

(54) **Sterilising hydrogels.**

(57) A method of simultaneously sterilising and incorporating a medicament into a hydrogel, which comprises applying a predetermined amount of medicament to the hydrogel which is then sealed within a water impermeable pack, heating the pack to a temperature such that water contained within the hydrogel is converted into steam and allowing the pack to cool, whereby the medicament is carried into the hydrogel by the steam and dispersed throughout its strucure and at the same time the hydrogel is sterilised.

0183441

84/28899

### Sterilising Hydrogels

This invention relates to a method of sterilising hydrogels.

Hydrogels, which are hydrophilic polymeric gels, insoluble in water, are commonly used as wound dressings. It is often desirable to incorporate medicaments into the structure of the gel but a uniform distribution of the medicament can be difficult to achieve. We have now found a simple way of carrying this out.

The present invention thus provides a method of simultaneously sterilising and incorporating a medicament into a hydrogel, which comprises applying a predetermined amount of medicament to the hydrogel which is then sealed within a water impermeable pack, heating the pack to a temperature such that water contained within the hydrogel is converted into steam and allowing the pack to cool, whereby the medicament is carried into the hydrogel by the steam and dispersed throughout its structure and at the same time the hydrogel is sterilised. Preferably the pack is heated to at least 100°C.

Various types of hydrogel can be used but the invention is particularly applicable to polyethyleneoxide based hydrogels, such as those described in U.S.P. 3,419,006. Other suitable hydrogels can include those based on agar/acrylamide copolymers and also those based on starch/acrylonitrile copolymers such as described in our U.K. Applications No. 83/18990 and 83/18991. Other hydrogels include those based on polyvinylpyrrolidene e.g. as described in EP 107376.

Preferably the hydrogel is in sheet form e.g. having a thickness of 0.1 to 10mm. especially 1 to 5mm. The degree of association of water contained in the hydrogel is sufficiently weak to allow water to be converted into steam and diffuse out under autoclave conditions.

The hydrogel sheet is usually covered on one or both sides by a polymer film. The film on at least one side of the sheet, is permeable to steam either by virtue of its own nature or by being suitably perforated. Such films are usually formed from a polyolefin and have a thickness, e.g. of 5 to 50 microns. preferably 10 to 40 microns.

A wide range of medicaments can be incorporated into the hydrogel e.g. water-soluble or lipid-soluble drugs either alone or with a solvent e.g. a water miscible solvent, and/or a suitable surfactant, the latter for instance to allow incorporation of lipophilic agents. Generally the drug is a biocide such as an antibiotic or other anti-bacterial agent e.g. chlorhexidine, povidone-iodine. cetrimide. silver sulphadiazine, etc.

The hydrogel is incorporated into a pack which usually comprises a laminate of various materials, the inner layer of which is a thermoplastic material, such as polyethylene or polypropylene, so that the packs can be heat sealed. Prior to sealing it is desirable to exclude air either by flushing with an inert gas or by removing it by vacuum.

The invention is illustrated in the following examples.

Example 1

Cross-linked polyethyleneoxide pre-gelled hydrogel sheet was covered on one side by 20 micron orientated polypropylene film, and on the other by 20 micron orientated polypropylene film, perforated so that it is steam permeable. The hydrogel was dosed with 30% by weight propylene glycol, and packed under vacuum in a laminated overpack. The whole was autoclaved at 115°C for 30 minutes. The resulting product was sterile and propylene glycol was homogenously distributed throughout the gel structure.

Example 2

Cross-linked polyethyleneoxide pre-gelled sheet was covered on one side only by a spun-bonded polypropylene sheet and dosed with 10% by weight povidone-iodine powder, and packed under vacuum in a laminated over-pack. After autoclaving at 115°C for 30 minutes, the resulting product was sterile and povidone-iodine was homogenously distributed throughout the gel structure.

Example 3

Hydrogel sheet formed by co-polymerisation of agar and acrylamide was covered on one side by 20 micron orientated polypropylene sheet and on the other side with a perforated 30 micron polyester film. Chlorhexidine acetate powder, (1% by weight) was added and the resulting product sealed in a laminated over-pack. After autoclaving at 115°C for 30 minutes, the chlorhexidine acetate was homogenously distributed throughout the gel structure, and the whole product was sterile.

CLAIMS

1) A method of simultaneously sterilising and incorporating a medicament into a hydrogel, which comprises applying a predetermined amount of medicament to the hydrogel which is then sealed within a water impermeable pack, heating the pack to a temperature such that water contained within the hydrogel is converted into steam and allowing the pack to cool, whereby the medicament is carried into the hydrogel by the steam and dispersed throughout its structure and at the same time the hydrogel is sterilised.

2) A method according to claim 1 wherein the pack is heated to at least 100°C.

3) A method according to claim 1 wherein the hydrogel is a polyethyleneoxide hydrogel.

## EUROPEAN SEARCH REPORT

European Patent Office

### DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| D,X | US-A-3 419 006 (P.A. KING) * Column 7, lines 30-51,69-75; column 8, lines 1-4 * | 1-3 | A 61 L 2/06 // A 61 L 15/03 A 61 K 9/70 |
| D,X | EP-A-0 107 376 (JOHNSON & JOHNSON) * Page 10, lines 30-37; page 11, lines 6-8 * | 1,2 | |
| D,X | EP-A-0 100 458 (SCHERING) * Examples 1-3; page 4, lines 1-12 * | 1,2 | |

|  | TECHNICAL FIELDS SEARCHED (Int. Cl.4) |
|---|---|
|  | A 61 L |

The present search report has been drawn up for all claims

| Place of search | Date of completion of the search | Examiner |
|---|---|---|
| THE HAGUE | 27-01-1986 | PELTRE CHR. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82